# EUROPEAN PATENT APPLICATION

(11) **EP 4 763 183 A1**
(43) Date of publication of application: **24.06.2026**
(21) Application number: 24856221.7
(22) Date of filing: 26.07.2024
(51) Int. Cl.: A61K 8/02, A61K 8/37, A61K 8/92, A61Q 1/02, A61Q 1/06

(54) **OILY SOLID COSMETIC AND HARDNESS IMPROVER FOR OILY SOLID COSMETIC**

(30) Priority: 18.08.2023 JP 2023133617
(71) Applicant: THE NISSHIN OILLIO GROUP, LTD., Tokyo 104-8285 (JP)
(72) Inventor: SHIBATA Masashi, Hachioji-shi, Tokyo 192-0982 (JP); TAKETANI Shunsuke, Yokohama-shi, Kanagawa 235-8558 (JP); OYAMA Keiichi, Yokohama-shi, Kanagawa 235-8558 (JP)
(74) Representative: Hasegawa, Kan
(86) International application number: PCT/JP2024/026800
(87) International publication number: WO 2025/041528

(57) **Abstract**

The present invention provides an oily solid cosmetic comprising component (1): one or two or more waxes selected from plant-based waxes that are solid at 25°C and animal-based waxes that are solid at 25°C; and component (3): an oligoester obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D), wherein the esterification reaction is performed using 0.65 to 0.8 moles of the component (B) and a total amount of 1.2 to 1.7 moles of the components (C) and (D) per mole of the component (A), a mass ratio of the component (C) to the component (D) used in the esterification reaction is component (C):component (D)=10:90 to 99.9:0.1, and the oligoester has an acid value of 5 mgKOH/g or less; a saponification value of 420 to 510 mgKOH/g; and a hydroxyl value of 60 mgKOH/g or less.

## Description

### TECHNICAL FIELD

The present invention relates to an oily solid cosmetic containing an oligoester.

### BACKGROUND ART

In recent years, from the viewpoints of safety and aesthetics, many naturally derived cosmetics have appeared on the market. In this market situation, consumer demand for naturally derived oily solid cosmetics has also increased.

For example, Patent Document 1 discloses an oily solid lip cosmetic that combines moldability and a good feel upon application by containing sunflower wax, a paste-like dimer acid ester, and a monoglycerin or polyglycerin ester. While the technology described in Patent Document 1 can combine moldability and a good feel upon application, it is difficult to achieve a glossy texture because it requires the inclusion of a large amount of paste oil. Furthermore, no consideration has been given to suppressing oil bleeds.

Patent Document 2 discloses a lipstick composition that contains natural wax, mineral oil, and castor oil, thereby providing excellent aesthetic performance and lip protection. However, although the composition provides excellent aesthetic performance and lip protection, it has the problem of poor oxidative stability due to the large amount of castor oil it contains.

Patent Document 3 discloses a technology for improving shape retention even with a small amount of wax by using selected rice bran wax, but rice bran wax that meets this performance requirement is difficult to obtain, making it impractical.

Meanwhile, oligoesters have been used in cosmetics for a long time due to their skin moisturizing effect, high safety, and excellent pigment dispersibility. A typical example of an oligoester incorporated into cosmetics is an oligoester that functions as a gelling or solidifying agent for liquid oil (Patent Document 4).

### Citation List

### Patent Documents

Patent Document 1: Japanese Patent No. 7072845
Patent Document 2: Japanese Unexamined Patent Application, First Publication No. H6-316509
Patent Document 3: Japanese Unexamined Patent Application, First Publication No. 2009-179564
Patent Document 4: Japanese Unexamined Patent Application, Fist Publication No. 7-126604

### SUMMARY OF INVENTION

### Technical Problem

An object of the present invention is to provide an oily solid cosmetic that contains a plant-based wax or an animal-based wax, has excellent shape retention, is effective in suppressing oil bleeds during use, and has a good feel upon application.

Another object of the present invention is to provide a hardness improver that has an excellent hardness improving effect when mixed with a plant-based wax or an animal-based wax.

### Solution to Problem

As a result of intensive research conducted by the present inventors to solve the above-mentioned problems, they discovered that an oligoester obtained by an esterification reaction between a glycerin, a dicarboxylic acid having 2 to 12 carbon atoms, and a specific combination of two or more fatty acids has excellent shape retention, excellent suppression of oily bleeds during use, and a good feel upon application, and also has excellent oxidation stability for an oily solid cosmetic composition containing a plant-based wax or an animal-based wax that is solid at 25°C, and thus completed the present invention.

That is, the present invention includes the following aspects.
[1] An oily solid cosmetic comprising:
   component (1): one or two or more waxes selected from plant-based waxes that are solid at 25°C and animal-based waxes that are solid at 25°C; and
   component (3): an oligoester obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D), wherein
   the esterification reaction is performed using 0.65 to 0.8 moles of the component (B) and a total amount of 1.2 to 1.7 moles of the components (C) and (D) per mole of the component (A),
   a mass ratio of the component (C) to the component (D) used in the esterification reaction is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, most preferably component (C):component (D)=50:50 to 70:30, and also preferably component (C):component (D)=10:90 to 70:30, and
   the oligoester has an acid value of 5 mgKOH/g or less, preferably 0 to 3 mgKOH/g, and more preferably 0 to 2 mgKOH/g; a saponification value of 420 to 510 mgKOH/g, preferably 420 to 490 mgKOH/g, and more preferably 430 to 490 mgKOH/g; and a hydroxyl value of 60 mgKOH/g or less, preferably 0 to 60 mgKOH/g, more preferably 0 to 40 mgKOH/g, even more preferably 0 to 35 mgKOH/g, and still more preferably 0 to 20 mgKOH/g.
[2] The oily solid cosmetic according to [1], wherein the component (1) is one or two or more waxes selected from the group consisting of a carnauba wax, a rice bran wax, a candelilla wax, a sunflower seed wax, and a beeswax.
[3] The oily solid cosmetic according to [1] or [2], wherein the plant-based wax is one or two or more selected from the group consisting of a carnauba wax, a rice bran wax, a candelilla wax, and a sunflower seed wax.
[4] The oily solid cosmetic according to any one of [1] to [3], wherein the oily solid cosmetic does not contain any hydrocarbon wax (excluding plant-based waxes containing naturally derived hydrocarbon components and animal-based waxes containing naturally derived hydrocarbon components).
[5] The oily solid cosmetic according to any one of [1] to [4], wherein the component (B) is a succinic acid.
[6] The oily solid cosmetic according to any one of [1] to [5], wherein the component (C) is a caprylic acid.
[7] The oily solid cosmetic according to any one of [1] to [6], wherein the component (D) is a capric acid.
[8] The oily solid cosmetic according to any one of [1] to [7], wherein the component (B) is a succinic acid, the component (C) is a caprylic acid, and the component (D) is a capric acid.
[9] The oily solid cosmetic according to any one of [1] to [8], further comprising:
   component (2): an oily component that is liquid or pasty at 20°C (excluding the component (3)).
[10] The oily solid cosmetic according to any one of [1] to [8], wherein
   a content of the component (1) is 5 to 30% by mass, preferably 5% by mass or more but less than 30% by mass, more preferably 10 to 30% by mass, more preferably 10% by mass or more but less than 30% by mass, and even more preferably 14 to 20% by mass, and a content of the component (3) is 10 to 90% by mass, preferably 20 to 90% by mass, and more preferably 20 to 60% by mass, relative to a total mass of the oily solid cosmetic.
[11] The oily solid cosmetic according to [9] or [10], wherein
   the content of the component (1) is 5 to 30% by mass, preferably 5% by mass or more but less than 30% by mass, more preferably 10 to 30% by mass, more preferably 10% by mass or more but less than 30% by mass, and even more preferably 14 to 20% by mass, the content of the component (2) is more than 0% by mass but not more than 60% by mass, preferably more than 0% by mass but not more than 50% by mass, and more preferably more than 0% by mass but not more than 40% by mass, and the content of the component (3) is 10 to 90% by mass, preferably 20 to 90% by mass, and more preferably 20 to 60% by mass, relative to the total mass of the oily solid cosmetic.
[12] The oily solid cosmetic according to any one of [9] to [11], wherein a mass ratio of the component (3) to a total amount of the component (2) and the component (3) ([amount (g) of component (3)]/([amount (g) of component (2)]+[amount (g) of the component (3)]) is 0.2 to 1.0.
[13] The oily solid cosmetic according to any one of [1] to [12], further comprising:
   component (4): one or two or more selected from the group consisting of a 1,2-hexanediol, a caprylyl glycol, an ethylhexylglycerin, and a phenoxyethanol.
[14] The oily solid cosmetic according to any one of [1] to [13], wherein the oily solid cosmetic is a stick-type cosmetic.
[15] A hardness improver for improving hardness of an oily solid cosmetic, comprising an oligoester obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D), wherein
   the esterification reaction is performed using 0.65 to 0.8 moles of the component (B) and a total amount of 1.2 to 1.7 moles of the components (C) and (D) per mole of the component (A),
   a mass ratio of the component (C) to the component (D) used in the esterification reaction is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, most preferably component (C):component (D)=50:50 to 70:30, and also preferably component (C):component (D)=10:90 to 70:30,
   the oligoester has an acid value of 5 mgKOH/g or less, preferably 0 to 3 mgKOH/g, and more preferably 0 to 2 mgKOH/g; a saponification value of 420 to 510 mgKOH/g, preferably 420 to 490 mgKOH/g, and more preferably 430 to 490 mgKOH/g; and a hydroxyl value of 60 mgKOH/g or less, preferably 0 to 60 mgKOH/g, more preferably 0 to 40 mgKOH/g, even more preferably 0 to 35 mgKOH/g, and still more preferably 0 to 20 mgKOH/g, and
   the oily solid cosmetic contains one or two or more waxes selected from plant-based waxes that are solid at 25°C and animal-based waxes that are solid at 25°C.
[16] Use of an oligoester as a hardness improver for improving hardness of an oily solid cosmetic, wherein
   the oligoester is obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D),
   the esterification reaction is performed using 0.65 to 0.8 moles of the component (B) and a total amount of 1.2 to 1.7 moles of the components (C) and (D) per mole of the component (A),
   a mass ratio of the component (C) to the component (D) used in the esterification reaction is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, most preferably component (C):component (D)=50:50 to 70:30, and also preferably component (C):component (D)=10:90 to 70:30,
   the oligoester has an acid value of 5 mgKOH/g or less, preferably 0 to 3 mgKOH/g, and more preferably 0 to 2 mgKOH/g; a saponification value of 420 to 510 mgKOH/g, preferably 420 to 490 mgKOH/g, and more preferably 430 to 490 mgKOH/g; and a hydroxyl value of 60 mgKOH/g or less, preferably 0 to 60 mgKOH/g, more preferably 0 to 40 mgKOH/g, even more preferably 0 to 35 mgKOH/g, and still more preferably 0 to 20 mgKOH/g, and
   the oily solid cosmetic contains one or two or more waxes selected from plant-based waxes that are solid at 25°C and animal-based waxes that are solid at 25°C. Advantageous Effects of Invention

According to the present invention, it is possible to provide an oily solid cosmetic that contains a plant-based wax or an animal-based wax, has excellent shape retention, is effective in suppressing oil bleeds during use, and has a good feel upon application.

Furthermore, according to the present invention, it is possible to provide a hardness improver that has an excellent hardness improving effect on one or two or more waxes selected from plant-based waxes that are solid at 25°C and animal-based waxes that are solid at 25°C.

### DESCRIPTION OF EMBODIMENTS

In the present invention and the present specification, a numerical range of "A or more and B or less (A and B are real numbers satisfying A<B)" may be expressed as "A to B". For example, when it is written as "1 to 10 parts by mass", it means a numerical range from 1 part by mass to 10 parts by mass that includes the lower limit (1 part by mass) and the upper limit (10 parts by mass), that is, "1 part by mass or more and 10 parts by mass or less".

### <Oily solid cosmetic>

An overview of the oily solid cosmetic of the present embodiment will be described.

The oily solid cosmetic of the present embodiment contains the following specific wax (component (1)) and specific oligoester (component (3)). It is also preferable that the oily solid cosmetic of the present embodiment contains the following component (2) in addition to the following components (1) and (3).

Component (1): One or two or more waxes selected from plant-based waxes that are solid at 25°C and animal-based waxes that are solid at 25°C.

Component (2): An oily component that is liquid or pasty at 20°C (excluding the component (3)).

Component (3): An oligoester obtained by an esterification reaction of component (A): a glycerin, component (B): a dicarboxylic acid having 2 to 12 carbon atoms, component (C): one or two or more fatty acids selected from the group consisting of a caprylic acid, an ethylhexanoic acid, an isononanoic acid, an isopalmitic acid, and an isostearic acid, and component (D): one or two or more fatty acids selected from linear saturated fatty acids having 10 to 24 carbon atoms, and having an acid value of 5 mgKOH/g or less, a saponification value of 420 to 510 mgKOH/g, and a hydroxyl value of 60 mgKOH/g or less.

### (Component (1))

The wax of the component (1) contained in the oily solid cosmetic of the present embodiment is one or two or more waxes selected from plant-based waxes that are solid at 25°C and animal-based waxes that are solid at 25°C. By incorporating plant-based waxes or animal-based waxes together with the specific oligoester of the component (3), an oily solid cosmetic that is excellent in all of shape retention, suppression of oil bleeding during use, and good feel upon application can be obtained. The wax of the component (1) contained in the oily solid cosmetic of the present embodiment may be one type, or two or more types.

The terms "plant-based wax" and "animal-based wax" refer to waxes derived from plants or animals, respectively. The oily solid cosmetic of the present embodiment preferably does not contain hydrocarbon waxes such as an ozokerite, a ceresin, a paraffin, a petrolatum, a microcrystalline wax, Fischer-Tropsch wax, a polyethylene wax, an ethylene-propylene copolymer, or the like. However, plant-based waxes containing naturally occurring hydrocarbon components and animal-based waxes containing naturally occurring hydrocarbon components are not included in the hydrocarbon waxes and can be used as the wax of the component (1). Examples of the plant-based wax containing naturally occurring hydrocarbon components include a candelilla wax hydrocarbon, or the like.

The wax of the component (1) is not particularly limited as long as it is a plant-based wax or an animal-based wax that is solid at 25°C and melts to a liquid at 120°C. Among the plant-based waxes and the animal-based waxes, the wax of the component (1) is preferably a wax having a melting point in the range of 50°C to 100°C, and more preferably a wax having a melting point in the range of 60°C to 90°C. By having a melting point in the above range, an oily solid cosmetic having improved shape retention, a greater effect in suppressing oil bleeds during use, and an extremely good feel upon application can be obtained.

The melting point of the wax used in the present invention and the present embodiment can be measured according to "71. Melting Point Determination Method, Method 2" of the Quasi-drug Ingredients Standards 2021. Specifically, the measurement is performed as follows. First, the sample (the wax whose melting point is to be measured) is melted at a low temperature and then drawn up to a height of approximately 10 mm into a capillary tube approximately 120 mm long with both ends open. The sample is then left at 10°C or below for 24 hours or ice-cooled for at least 2 hours. Next, the sample is attached to an immersion thermometer or a fully submerged thermometer so that the sample is positioned outside the center of the mercury bulb. The sample is then placed in a 250 mL beaker containing water, with the top end of the sample held 10 mm below the water surface. The water in the beaker is heated while constantly being stirred. When the temperature reaches 5°C below the predicted melting point, heating is continued at a rate of 1°C per minute. The melting point is determined as the temperature at which the sample floats up in the capillary tube.

Examples of the plant-based wax having a melting point of 50°C to 100°C that can be used as the wax of the component (1) include a carnauba wax, a rice bran wax, a candelilla wax, a candelilla wax hydrocarbon, a sunflower seed wax, a hydrogenated jojoba oil, a wood wax, and the like. Examples of the animal-based wax having a melting point of 50°C to 100°C include a beeswax, a spermaceti and the like, and one or two or more of these can be used. Of these, the wax of the component (1) is preferably one or, two or, three or, four or more selected from a carnauba wax, a rice bran wax, a candelilla wax, a sunflower seed wax, and a beeswax.

The content of the component (1) in the oily solid cosmetic of the present embodiment is not particularly limited and can be set appropriately taking into consideration the desired quality characteristics of the oily solid cosmetic. For example, the content (mass ratio) of the component (1) relative to the entire oily solid cosmetic is preferably 5 to 30% by mass, more preferably 5 to less than 30% by mass, more preferably 10 to 30% by mass, more preferably 10 to less than 30% by mass, and even more preferably 14 to 20% by mass. By having the content of the component (1) relative to the entire oily solid cosmetic within the above range, it becomes easy to obtain an oily solid cosmetic that has sufficient hardness and that can be applied smoothly.

### (Component (3))

The oligoester of the component (3) contained in the oily solid cosmetic of the present embodiment is an oligoester obtained by esterification of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D). The esterification reaction to obtain the oligoester of the component (3) is performed by esterifying 0.65 to 0.8 moles of the component (B) and a total amount of 1.2 to 1.7 moles of the components (C) and (D) per mole of the component (A), and the mass ratio of the component (C) to the component (D) used in the esterification reaction is component (C):component (D)=10:90 to 99.9:0.1, preferably component (C):component (D)=10:90 to 99:1, more preferably component (C):component (D)=25:75 to 99 :1, even more preferably component (C):component (D)=40:60 to 99:1, still more preferably component (C):component (D)=40:60 to 70:30, and most preferably component (C):component (D)=50:50 to 70:30, and it is also preferable that component (C):component (D)=10:90 to 70:30.

The oligoester of the component (3) has an acid value of 5 mgKOH/g or less, preferably 0 to 3 mgKOH/g, and more preferably 0 to 2 mgKOH/g; has a saponification value of 420 to 510 mgKOH/g, preferably 420 to 490 mgKOH/g, and more preferably 430 to 490 mgKOH/g; and has a hydroxyl value of 60 mgKOH/g or less, preferably 0 to 60 mgKOH/g, more preferably 0 to 40 mgKOH/g, even more preferably 0 to 35 mgKOH/g, and even more preferably 0 to 20 mgKOH/g.

The oligoester of the component (3) has the effect of improving the hardness of the oily solid cosmetic (oily solid cosmetic hardness-improving effect). Therefore, the oily solid cosmetic of the present embodiment containing this oligoester has a sufficiently higher hardness and can improve shape retention compared to an oily solid cosmetic that does not contain this oligoester.

The oligoester of the component (3) is liquid at 20°C.

Furthermore, the oligoester of the component (3) has good low-temperature stability and is inhibited from crystallizing even at low temperatures. As a result, the oily solid cosmetic of the present embodiment containing this oligoester is inhibited from crystallizing even at low temperatures and is maintained stably.

The oligoester of the component (3) has excellent pigment dispersibility, and therefore, by adding the oligoester of the component (3) to an oily solid cosmetic containing a pigment, the dispersibility of the pigment can be improved, and the pigment can be dispersed uniformly throughout the oily solid cosmetic.

The oligoester of the component (3) contained in the oily solid cosmetic of the present embodiment may be one type, or two or more types.

The component (B), which is used as a raw material for the oligoester of the component (3), is a dicarboxylic acid having 2 to 12 carbon atoms. Examples of the dicarboxylic acid having 2 to 12 carbon atoms include an oxalic acid (ethanedioic acid), a malonic acid (propanedioic acid), a succinic acid (butanedioic acid), a glutaric acid (pentanedioic acid), an adipic acid (xanedioic acid), a pimelic acid (heptanedioic acid), a veric acid (octanedioic acid), an azelaic acid (nonanedioic acid), a sebacic acid (decanedioic acid), a phthalic acid (benzene-1,2-dicarboxylic acid), an isophthalic acid (benzene-1,3-carboxylic acid), a terephthalic acid (benzene-1,4-dicarboxylic acid), and the like. A succinic acid is particularly preferable as the component (B), which is used as a raw material for the oligoester of the component (3), from the viewpoint of further improving hardness.

The component (C), which is used as a raw material for the oligoester of the component (3), is one or two or more fatty acids selected from the group consisting of a caprylic acid, an ethylhexanoic acid, an isononanoic acid, an isopalmitic acid, and an isostearic acid. A caprylic acid is particularly preferable as the component (C), which is used as a raw material for the oligoester of the component (3). Furthermore, the oligoester of the component (3) has better color and odor than oligoesters obtained by esterification of unsaturated fatty acids with components (A) and (B).

The component (D), which is used as a raw material for the oligoester of the component (3), is one or two or more fatty acids selected from linear saturated fatty acids having 10 to 24 carbon atoms. Specific examples of such fatty acids include a capric acid (n-decanoic acid), a lauric acid, a myristic acid, a palmitic acid, a stearic acid, and a behenic acid. A capric acid is particularly preferable as the component (D), which is used as a raw material for the oligoester of the component (3).

The acid value of the oligoester of the component (3) is 5 mgKOH/g or less (0 to 5 mgKOH/g). **If** the acid value is higher than 5 mgKOH/g, there is a risk of odor generation. Since the oligoester of the component (3) has an acid value of 5 mgKOH/g or less, it does not have a distinctive odor. Therefore, the oily solid cosmetic of the present embodiment can be provided with excellent shape retention due to the oligoester of the component (3) without impairing the desired fragrance quality.

The acid value of the oligoester of the component (3) can be adjusted to fall within the above range by sampling the reaction product during the esterification reaction, measuring its acid value, and terminating the esterification reaction when the desired acid value is reached.

The acid value of the oligoester of the component (3) is 5 mgKOH/g or less, preferably 0 to 3 mgKOH/g, and more preferably 0 to 2 mgKOH/g.

The saponification value of the oligoester of the component (3) is 420 to 510 mgKOH/g, preferably 420 to 490 mgKOH/g, and more preferably 430 to 490 mgKOH/g. When the saponification value is within this range, the oligoester exhibits a hardness-improving effect.

The saponification value of the oligoester of the component (3) can be adjusted by the amounts charged in the esterification reaction.

The hydroxyl value of the oligoester of the component (3) is 60 mgKOH/g or less, preferably 0 to 60 mgKOH/g, more preferably 0 to 40 mgKOH/g, even more preferably 0 to 35 mgKOH/g, and still more preferably 0 to 20 mgKOH/g. If the hydroxyl value is too high, wax solubility may be poor. By having a hydroxyl value of 60 mgKOH/g or less, the oligoester of the component (3) has good wax solubility.

The hydroxyl value of the oligoester of the component (3) can be adjusted by the amounts charged in the esterification reaction.

These components (A) to (D) can be commercially available products.

Glycerin and linear saturated fatty acids derived from plants are commonly available, and succinic acid is also mass-produced industrially by fermentation. Therefore, by using a succinic acid as the component (B), the oligoester of the component (3) can be produced using a combination of inexpensive raw materials. In particular, from the viewpoints of environmental impact and sustainability of supply, it is preferable to use a glycerin as the component (A), a succinic acid as the component (B), a caprylic acid which is a linear saturated fatty acid as the component (C), and a capric acid as the component (D), as raw materials for the oligoester of the component (3).

The degree of crosslinking and the number of side chains of the oligoester of the component (3) can be adjusted by changing the amounts of raw materials charged in the esterification reaction, thereby obtaining an oligoester with various effects. Specifically, the degree of crosslinking of the obtained oligoester of the component (3) can be adjusted by changing the amounts of the components (A) and (B) charged. Furthermore, the degree of crosslinking and the number of added side chains can be adjusted by changing the amount of fatty acid charged as the component (C).

In the esterification reaction for producing the oligoester of the component (3), 0.65 to 0.8 moles of the component (B) and a total amount of 1.2 to 1.7 moles of the components (C) and (D) per mole of the component (A) are subjected to an esterification reaction. The mass ratio of the component (C) to the component (D) is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, most preferably component (C):component (D)=50:50 to 70:30, and also preferably component (C):component (D)=10:90 to 70:30.

If the amount of the component (B) charged per mole of the component (A) is less than 0.65 moles, the resulting oligoester may not have the effect of improving the hardness of the oily solid cosmetic. On the other hand, if more than 0.8 moles of the component (B) are used, crosslinking between the component (A) and the component (B) may proceed too far, causing the resulting oligoester to become cloudy.

If the amount of the component (C) charged per mole of the component (A) exceeds 1.7 moles, transesterification may occur, and synthesis may not proceed to the desired degree of crosslinking. On the other hand, if the amount of the component (C) charged is less than 1.2 moles, the amount of unreacted hydroxyl groups may increase, and the wax solubility of the resulting oligoester may be poor.

The mass ratio of the components (C) and (D) charged is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, and most preferably component (C):component (D)=50:50 to 70:30, and also preferably component (C):component (D)=10:90 to 70:30. In the mass ratio of the components (C) and (D) charged, the mass ratio of the component (D) ([mass of the component (D)]/([mass of the component (C)]+[mass of the component (D)])) may be less than 0.1. On the other hand, when the mass ratio of the component (C) ([mass of the component (C)]/([mass of the component (C)]+[mass of the component (D)])) in the mass ratio of the components (C) and (D) charged is 8 (component (C):component (D)=8:92) or less, the obtained oligoester is prone to crystallization in a low-temperature environment and has poor low-temperature stability, which is not preferable.

The oligoester of the component (3) can be produced, for example, by charging 0.65 to 0.8 moles of the component (B) and a total amount of 1.2 to 1.7 moles of the components (C) and (D) per mole of the component (A), and reacting them at a temperature of 180 to 240°C in the presence or absence of a catalyst. The catalyst can be an acid, alkali, or other catalyst known per se in the field of organic chemistry, which is used in the esterification reaction of alcohols and fatty acids. The esterification reaction can be carried out in a solvent that does not adversely affect the esterification of alcohols and fatty acids, or it can be carried out without a solvent. The solvent can be a solvent known per se in the field of organic chemistry, which is used in the esterification reaction of alcohols and fatty acids.

The reaction time for the esterification reaction to produce the oligoester of the component (3) can usually be 20 to 30 hours. The reaction time may be 20 hours or less or 30 hours or more, depending on the raw material used (linear or branched), the presence or absence of a catalyst, the esterification temperature, the amount of excess acid, and other factors. After completion of the reaction, if a catalyst was used, it may be removed by filtration, adsorption, or the like.

By subjecting the components (A) to (D) to an esterification reaction, a reaction product containing an oligoester of the component (3) can be obtained. Purification of the reaction product by conventional purification methods can yield a purified oligoester. For example, the reaction product of the esterification reaction can be purified by removing excess unreacted raw materials by distillation. Furthermore, if it is desired to improve the color, etc., of the esterified product, the color can be improved by conventional decolorization treatment.

The content of the component (3) in the oily solid cosmetic of the present embodiment is not particularly limited, as long as it is an amount that exhibits the hardness-improving effect of the oligoester of the component (3). For example, the content of the component (3) relative to the entire oily solid cosmetic is preferably 10 to 90% by mass, more preferably 20 to 90% by mass, and even more preferably 20 to 60% by mass.

### (Component (2))

The oily solid cosmetic of the present embodiment may contain, in addition to the component (1) and the component (3), an oily component that is liquid or pasty at 20°C as the component (2).

When the component (2) is contained, the oily component of the component (2) is not particularly limited as long as it is an oily substance other than the component (3), and can be appropriately selected from various oily components that are generally blended into cosmetics and that are liquid or pasty at 20°C. The oily component of the component (2) contained in the oily solid cosmetic of the present embodiment may be one type, or two or more types.

Examples of the oily component of the component (2) that is liquid or pasty at 20°C include hydrocarbons, fatty acid esters, triglycerides, fatty acids, higher alcohols, silicone oils, fluorine-based oils, and derivatives thereof, and one or two or more of these can be used. Specific examples thereof include castor oil, olive oil, avocado oil, palm oil, cocoa oil, liquid paraffin, liquid branched paraffin, petrolatum, squalane, hydrogenated polyisobutene, hydrogenated polydecene, dicaprylic/capric propanediol, neopentyl glycol dicaprate, polyglyceryl-6 octacaprylate, tricaprylic/capric triglyceride, triethylhexanoin, butyl stearate, ethylhexyl palmitate, coco-caprylic/capric acid, caprylyl caprylate/capric acid, octadecyl myristate ctyldodecyl, isopropyl myristate, isopropyl lanolate, hexyl lanolate, diisopropyl adipate, diisopropyl sebacate, isotridecyl isononanoate, isononyl isononanoate, polyglyceryl decaisostearate, 2-octyldodecanol, diisostearyl malate, polyglyceryl-2 triisostearate, polyglyceryl-2 diisostearate, dipentaerythrityl pentaisostearate, dipentaerythrityl tetraisostearate, tetraisostearin pentaerythrityl triisostearate, trimethylolpropane triisostearate, dipentaerythrityl hexahydroxystearate/hexastearate/hexarosinate, cholesteryl hydroxystearate, phytosteryl macadamia seed oil fatty acid, bis(behenyl/isostearyl/phytosteryl) dimer dilinoleyl dimer dilinoleate, phytosteryl/octyldodecyl/behenyl lauroyl glutamate, glyceryl ethylhexanoate/stearate/adipic acid, oleyl alcohol, dimethyl polyglyceryl stearate polysiloxane, methylphenylpolysiloxane, dimethylcyclopolysiloxane, methylhydrogenpolysiloxane, perfluoropolyether, di(phytosteryl/octyldodecyl) lauroyl glutamate, dipentaerythrityl hexahydroxystearate, dipentaerythrityl tetra(hydroxystearate/isostearate), dipentaerythrityl tripolyhydroxystearate, mineral oil, cetyl ethylhexanoate, phenyl trimethicone, cyclopentasiloxane, isododecane, and the like.

The oily component of the component (2) contained in the oily solid cosmetic of the present embodiment is preferably one or two or more selected from a castor oil, diisostearyl malate, polyglyceryl-2 triisostearate, polyglyceryl-10 decaisostearate, tri(caprylic/capric)glyceryl, a macadamia seed oil, glyceryl (ethylhexanoate/stearate/adipic), and di(caprylic/capric)propanediol; more preferably one or two or more selected from polyglyceryl-2 triisostearate, polyglyceryl-10 decaisostearate, glyceryl tri(caprylic/capric acid), and a macadamia seed oil; and even more preferably one or two or more selected from polyglyceryl-2 triisostearate, polyglyceryl-10 decaisostearate, glyceryl tri(caprylic/capric acid), and a macadamia seed oil.

The content of the component (2) in the oily solid cosmetic of the present embodiment is not particularly limited, as long as it is a content that allows the hardness-improving effect of the oligoester of the component (3) to be exhibited, and can be set appropriately in consideration of the desired quality characteristics of the oily solid cosmetic. When the oily solid cosmetic of the present embodiment contains the component (2), for example, the content of the component (2) relative to the entire oily solid cosmetic is preferably more than 0% by mass and not more than 60% by mass, more preferably more than 0% by mass and not more than 50% by mass, and even more preferably more than 0% by mass and not more than 40% by mass.

### (Component (4))

The oily solid cosmetic of the present embodiment may also contain the following component (4) in addition to the component (1) and the component (3), or in addition to the component (1), the component (2), and the component (3), or in addition to the component (1), the component (2), the component (3), and an ultraviolet absorber described below. These alcohols are alcohols that are generally added to cosmetics as preservatives.

Component (4): One or two or more selected from the group consisting of 1,2-hexanediol, caprylyl glycol, ethylhexylglycerin, and phenoxyethanol.

The content of the component (4) in the oily solid cosmetic of the present embodiment is not particularly limited, as long as it is a content that allows the hardness-improving effect of the oligoester of the component (3) to be exhibited, and can be set appropriately in consideration of the desired quality characteristics of the oily solid cosmetic. For example, the content of the component (4) relative to the entire oily solid cosmetic is preferably more than 0% by mass and not more than 5.0% by mass, more preferably more than 0% by mass and not more than 2.0% by mass, and even more preferably more than 0% by mass and not more than 1.0% by mass.

In the oily solid cosmetic of the present embodiment, the content of the component (1) relative to the entire oily solid cosmetic is 5 to 30% by mass, preferably 10 to 30% by mass, and more preferably 14 to 20% by mass; the content of the component (2) relative to the entire oily solid cosmetic is 0 to 60% by mass, preferably 0 to 50% by mass, and more preferably 0 to 40% by mass; and the content of the component (3) relative to the entire oily solid cosmetic is 10 to 90% by mass, preferably 20 to 90% by mass, and more preferably 20 to 60% by mass; the mass ratio of the component (3) to the total amount of the component (2) and the component (3) ([amount (mass) of the component (3)]/([amount (mass) of the component (2)]+[amount (mass) of the component (3)]) is 0.2 to 1.0; and the content of the component (4) relative to the entire oily solid cosmetic is 0 to 5.0 mass%, preferably 0 to 2.0 mass%, more preferably 0 to 1.0 mass%. Note that "a content of the component (X) of 0 mass%" means that the component (X) is not contained.

### (Ultraviolet absorber)

The oily solid cosmetic of the present embodiment preferably contains an ultraviolet absorber in addition to the component (1) and the component (3), or in addition to the component (1), the component (2), and the component (3), or in addition to the component (1), the component (2), the component (3), and the component (4).

Examples of the ultraviolet absorber contained in the oily solid cosmetic of the present embodiment include, but are not limited to, benzoic acid-based ultraviolet absorbers such as para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, or the like; anthranilic acid-based ultraviolet absorbers such as homomenthyl-N-acetylanthranilate, or the like; salicylic acid-based ultraviolet absorbers such as amyl salicylate, menthyl salicylate, homomenthyl salicylate, octyl salicylate, phenyl salicylate, benzyl salicylate, p-isopropanol phenyl salicylate, or the like; cinnamic acid-based UV absorbers such as octyl cinnamate, ethyl 4-isopropyl cinnamate, methyl 2,5-diisopropyl cinnamate, ethyl 2,4-diisopropyl cinnamate, methyl 2,4-diisopropyl cinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano No-β-phenyl cinnamate, glyceryl mono-2-ethylhexanoyl-di-para-methoxycinnamate, or the like; benzophenone-based UV absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenone nyl-benzophenone-2-carboxylate, 2-hydroxy-4-n-octoxybenzophenone, 4-hydroxy-3-carboxybenzophenone, or the like; 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t-butyldibenzoylmethane, 5-(3,3-dimethyl-2-norbornylidene)-3-pentan-2-one, 2,4,6-trianilino-p-(carbo-2'-ethylhexyl-1'-oxy)1,3,5-triazine, 4-tent-butyl-4'-methoxydibenzoylmethane, ethylaminohydroxybenzoylhexyl benzoate, ethylhexyl triazine, t-butylmethoxydibenzoylmethane, bisethylhexyloxyphenol methoxyphenyl triazine, diethylhexylbutamido triazone, oxybenzone-3, and the like. One type of the ultraviolet absorber may be used alone, or two or more types may be used in combination.

The content of the ultraviolet absorber in the oily solid cosmetic of the present embodiment is not particularly limited as long as it is a content that allows the hardness-improving effect of the oligoester of the component (3) to be exhibited, and can be appropriately set in consideration of the desired quality characteristics of the oily solid cosmetic. For example, the content of the ultraviolet absorber relative to the entire oily solid cosmetic is preferably more than 0% by mass and not more than 5.0% by mass, more preferably more than 0% by mass and not more than 2.0% by mass, and even more preferably more than 0% by mass and not more than 1.0% by mass.

### (Powder)

The oily solid cosmetic of the present embodiment may contain a powder in addition to the component (1) and the component (3), or in addition to the component (1), the component (2), and the component (3), or in addition to the component (1), the component (2), the component (3), and the component (4). The oligoester of the component (3) has good pigment dispersibility, and therefore, by blending a powder into the oily solid cosmetic of the present embodiment, an oily solid cosmetic in which the powder is uniformly dispersed throughout the cosmetic can be obtained.

As the powder, extender pigments, color pigments, and pearl pigments can be used.

Examples of the extender pigments include inorganic pigments such as silicic acid, silicic anhydride, magnesium silicate, talc, sericite, mica, kaolin, clay, bentonite, bismuth oxychloride, zirconium oxide, magnesium oxide, zinc oxide, aluminum oxide, calcium sulfate, barium sulfate, magnesium sulfate, calcium carbonate, and magnesium carbonate, and composite powders thereof; organic powders such as polyamide, polyester, polypropylene, polystyrene, polyurethane, nylon, silicone resin, vinyl resin, urea resin, phenolic resin, silicon resin, acrylic resin, melamine resin, epoxy resin, polycarbonate resin, divinylbenzene-styrene copolymer, silk powder, cellulose, Nε-lauroyl-L-lysine, long-chain alkyl phosphate metal salt, N-mono long-chain alkyl acyl basic amino acid, and metal soap, and composite powders thereof; composite powders of the above inorganic and organic powders; and the like. The particle shape of these powders may be any shape, such as spherical, plate-like, acicular, granular, irregular, or the like.

Examples of the coloring pigments include metal oxides such as titanium oxide, zinc oxide, yellow iron oxide, red iron oxide, black iron oxide, Prussian blue, ultramarine, chromium oxide, and chromium hydroxide; metal complexes such as manganese violet and cobalt titanate; inorganic pigments such as carbon black; organic pigments such as tar-based dyes and lake pigments; natural pigments such as carmine; and the like.

As the pearl pigment, there can be used pearl pigments in which mica, synthetic phlogopite, or the like is coated with a colorant such as titanium oxide, iron oxide, silicon oxide, Prussian blue, chromium oxide, carmine, organic pigments, and the like. These powders may be subjected to various surface treatments such as water repellency treatment, water/oil repellency treatment, or the like by a conventional method before use.

By incorporating a powder having an ultraviolet ray shielding effect, such as a metal oxide powder, it is also possible to produce a sunscreen cosmetic (a cosmetic for suppressing sunburn). The metal oxide powder having an ultraviolet ray shielding effect is not particularly limited, and examples thereof include titanium oxide, zinc oxide, zirconium oxide, cerium oxide, and the like, and one or two or more of these can be used.

The shape of the metal oxide powder having the UV-shielding effect is not particularly limited, and examples thereof include spherical, needle-like, spindle-like, plate-like, flaky shapes, and the like. The powder may be treated with an inorganic compound such as silica or alumina to reduce its surface activity. Furthermore, it is more preferable for the metal oxide powder to have an average particle size of 10 to 100 nm, since this prevents the powder from appearing white when applied to the skin.

The oily solid cosmetic of the present embodiment may contain one type of powder, or two or more types. The content of the powder in the oily solid cosmetic of the present embodiment is not particularly limited, as long as it is a content that allows the hardness-improving effect of the oligoester of the component (3) to be exhibited, and can be set appropriately taking into consideration the desired quality characteristics of the oily solid cosmetic. For example, the content of the powder relative to the entire oily solid cosmetic (total mass) is preferably 0 to 50% by mass, more preferably 0 to 40% by mass, and even more preferably 0 to 30% by mass.

### (Other additives)

The oily solid cosmetic of the present embodiment may contain additives other than the components (1) to (4), the UV absorbers, and the powders, as long as the effect of the oligoester of the component (3) is not impaired. One or two or more of these additives may be appropriately selected from known additives used in cosmetics, hygiene products, pharmaceuticals, etc. Specific examples of such additives include gelling agents (excluding the component (1)), surfactants, antioxidants, antioxidant aids, preservatives, alcohols (excluding the component (4)), water-soluble polymers, pH adjusters, inorganic salts or organic acid salts, chelating agents, vitamins, organic solvents, fragrances, various extracts, UV scattering agents, and the like.

Examples of the gelling agents contained in the oily solid cosmetic of the present embodiment include behenyl behenate, glyceryl tribehenate, dextrin ethylhexanoate, dextrin palmitate, dextrin myristate, dextrin isostearate, inulin palmitate, inulin stearate, higher fatty acids, metal soaps of higher fatty acids, silica silylate, dibutyl ethylhexanoyl glutamide, dibutyl lauroyl glutamide, and the like, which may be used alone or in combination of two or more.

Examples of the surfactants contained in the oily solid cosmetic of the present embodiment include a nonionic surfactant, anionic surfactant, cationic surfactant, amphoteric surfactant, and the like, which may be used alone or in combination of two or more.

Examples of the nonionic surfactants include monoglycerides, sorbitan fatty acid esters, sucrose fatty acid esters, polyglycerin fatty acid esters, alkanolamides, amine oxides, polyoxyethylene alkyl ethers, polyethylene glycol fatty acid esters, polyoxyethylene sorbitan fatty acid esters, polyoxyethylene glycerin fatty acid esters, polyoxyethylene propylene glycol mono-fatty acid esters, polyoxyethylene hydrogenated castor oil, polyoxyethylene fatty acid amides, polyoxyethylene alkylamines, alkylsaccharides, α-monoalkylglyceryl ethers, dimethylpolysiloxane-polyoxyalkylene copolymers, dimethylpolysiloxane-monoalkylglyceryl ether copolymers, sorbitan sesquiisostearate, and the like.

Examples of the anionic surfactants include alkylbenzene sulfonates, alkylnaphthalene sulfonates, polyoxyethylene alkyl ether sulfates, polyoxyethylene lauryl ether phosphates, and the like.

Examples of the cationic surfactants include primary, secondary and tertiary amine salts, quaternary ammonium salts having an aliphatic hydrocarbon group, and the like.

Examples of the amphoteric surfactants include sodium β-laurylaminopropionate, lauryldimethylaminoacetic acid betaine, 2-alkyl-N-carboxymethyl-N-hydroxyethylimidazolinium betaine, and the like.

Examples of other surfactants that can be used include lecithins, soybean saponin, and the like. Examples of the lecithins include lecithin, hydrogenated lecithin, hydroxide lecithin, lysolecithin, hydrogenated lysolecithin, and the like. Examples of the hydrogenated lecithins include hydrogenated soybean phospholipid, hydrogenated rapeseed phospholipid, hydrogenated egg yolk phospholipid, and the like.

Among the surfactants contained in the oily solid cosmetic of the present embodiment, nonionic surfactants such as polyoxyethylene-modified silicone, polyglycerin fatty acid ester, polyoxyethylene alkyl, polyoxyethylene fatty acid ester, and polyoxyethylene hydrogenated castor oil are preferable.

The antioxidant contained in the oily solid cosmetic of the present embodiment is not particularly limited, and examples thereof include oil-soluble vitamin C derivatives, tocopherols and their derivatives and salts, dibutylhydroxytoluene, butylhydroxyanisole, and gallic acid esters, and the like. The antioxidants may be used alone or in combination of two or more.

The antioxidant aid contained in the oily solid cosmetic of the present embodiment is not particularly limited, and examples thereof include phosphoric acid, citric acid, maleic acid, malonic acid, succinic acid, fumaric acid, cephalin, hexametaphosphate, phytic acid, ethylenediaminetetraacetic acid, and the like. The antioxidant aids may be used alone or in combination of two or more.

The preservative contained in the oily solid cosmetic of the present embodiment is not particularly limited, and examples thereof include methylparaben, ethylparaben, butylparaben, and the like. The preservatives may be used alone or in combination of two or more.

The alcohol contained in the oily solid cosmetic of the present embodiment is not particularly limited, and may be a monohydric alcohol or a polyhydric alcohol.

The monohydric alcohol may a lower alcohol or a higher alcohol. Examples of the lower alcohols include methanol, ethanol, propanol, isopropanol, isobutyl alcohol, and t-butyl alcohol. Examples of the higher alcohols include cetanol (cetyl alcohol, palmityl alcohol), stearyl alcohol (octadecyl alcohol), isostearyl alcohol (isooctadecanol), oleyl alcohol, cetostearyl alcohol, octyldodecanol, decyltetradecanol, hexyldecanol, behenyl alcohol, lauryl alcohol, lanolin alcohol, and hydrogenated lanolin alcohol. The monohydric alcohols may be used alone or in combination of two or more.

Examples of the polyhydric alcohols include propylene glycol (1,2-propanediol), 1,3-propanediol, 1,3-butylene glycol (1,3-butanediol), pentylene glycol (1,2-pentanediol), neopentylene glycol (2,2-dimethyl-1,3-propanediol), isoprene glycol (3-methyl-1,3-butanediol), dipropylene glycol, glycerin, diglycerin, polyglycerin, polyethylene glycol, pentaerythritol, dipentaerythritol, sorbitol, sorbitan, and the like. The polyhydric alcohols may be used alone or in combination of two or more.

The water-soluble thickener may be a natural water-soluble polymer, a semi-synthetic water-soluble polymer, or a synthetic water-soluble polymer. The water-soluble thickener contained in the oily solid cosmetic of the present embodiment may be used alone or in combination of two or more types.

Examples of the natural water-soluble polymers include plant-based polymers such as agar, glucomannan, gum arabic, tragacanth gum, galactan, guar gum, carob gum, karaya gum, carrageenan, pectin, quince seed (quince), algae colloid (cassow extract), starch (rice, corn, potato, wheat), or the like; microbial-based polymers such as xanthan gum, dextran, succinoglucan, pullulan, or the like; and animal-based polymers such as collagen, casein, albumin, gelatin, or the like.

Examples of the semi-synthetic water-soluble polymers include starch-based polymers such as carboxymethyl starch, methylhydroxypropyl starch, or the like; cellulose-based polymers such as methyl cellulose, nitrocellulose, methylhydroxypropyl cellulose, sodium cellulose sulfate, hydroxypropyl cellulose, carboxymethyl cellulose, sodium carboxymethyl cellulose, crystalline cellulose, cellulose powder, or the like; and alginic acid-based polymers such as sodium alginate, propylene glycol alginate, or the like; and the like.

Examples of the synthetic water-soluble polymers include vinyl polymers such as polyvinyl alcohol, polyvinyl methyl ether, polyvinylpyrrolidone, carboxyvinyl polymers, or the like; polyoxyethylene polymers such as polyethylene glycol 20,000, 40,000, 60,000, or the like; polyoxyethylene-polyoxypropylene copolymer polymers; acrylic polymers such as sodium polyacrylate, polyethyl acrylate, polyacrylamide, or the like; polyethyleneimine; cationic polymers; and the like.

Examples of pH adjusters include edetic acid, edetate disodium, citric acid, sodium citrate, sodium hydroxide, potassium hydroxide, triethanolamine, and the like. The pH adjusters may be used alone or in combination of two or more.

Examples of the inorganic salts include sodium chloride, potassium chloride, magnesium chloride, sodium sulfate, potassium sulfate, magnesium sulfate, and the like. Examples of the organic acid salts include citric acid, malic acid, tartaric acid, and salts thereof, ascorbic acid and salts thereof, ascorbic acid derivatives and salts thereof, and the like.

Examples of the chelating agent include disodium edetate, edetate salts, hydroxyethanediphosphonic acid, and the like. The chelating agents may be used alone or in combination of two or more.

Examples of the vitamins include vitamin A, vitamin B1, vitamin B2, vitamin B6, vitamin E, vitamin K and derivatives thereof, pantothenic acid and derivatives thereof, biotin, and the like.

Examples of the extracts include plant extracts such as aloe vera, witch hazel, hamamelis, cucumber, lemon, lavender, rose, and the like.

### (Method for producing oily solid cosmetic)

The oily solid cosmetic of the present embodiment can be produced, for example, by heating and mixing the oligoester of the component (3), the wax of the component (1), and other ingredients until homogeneous, and then filling the resulting liquid or paste-like mixture into a container for forming a desired shape and cooling it. The raw material ingredients may be mixed all at once or sequentially.

The oily solid cosmetic of the present embodiment containing the component (2) can be produced, for example, by heating and mixing the oligoester of the component (3), the wax of the component (1), the oily component of the component (2), and other components until homogeneous, and then filling the resulting liquid or paste-like mixture into a container for forming a desired shape and cooling it. The raw material components may be mixed all at once or sequentially.

The shape of the oily solid cosmetic of the present embodiment is not particularly limited, and it can be molded into various shapes, such as a rod shape, a plate shape, or a shape formed by pouring into a dish-like object.

The oily solid cosmetic of the present embodiment is not particularly limited, and examples thereof include makeup cosmetics such as lipstick, foundation, concealer, blush, eyebrow makeup, eyeshadow, eyeliner, or the like; body care cosmetics such as UV care products, deodorant products, body skin care products, body fragrance products, body cleansing products, or the like; cleansing products such as cleansing balms, or the like; skin care cosmetics such as whitening cosmetics, UV protection cosmetics, anti-aging products, skin care balms, or the like; hair care cosmetics such as hair styling wax, hair sticks, hair treatments, one-day hair color, hair gloss, or the like; fragrance cosmetics such as perfumes, eau de toilette, eau de cologne, eau de parfum, stick-type solid perfumes, or the like; topical skin care products such as ointments, sunscreens, or the like; and the like. In addition to lipstick, the lip cosmetics can also be used as primer cosmetics and finishing gloss cosmetics known as lip balms, lip glosses, lip colors, and the like.

The oily solid cosmetic of the present embodiment is particularly preferably a stick-type cosmetic such as lipstick or lip gloss, because it has excellent shape retention.

### <Hardness improver>

The oligoester of the component (3) can improve the hardness of an oily solid cosmetic containing one or two or more waxes selected from the above-mentioned plant-based waxes that are solid at 25°C and animal-based waxes that are solid at 25°C. Therefore, the oligoester of the component (3) can be used as a hardness improver for oily solid cosmetic compositions.

Furthermore, even when an oily solid cosmetic contains the above-mentioned oil component that is liquid or pasty at 20°C, the hardness can be improved by blending the oligoester of the component (3). Therefore, the oligoester of the component (3) can be used as a hardness improver for an oily solid cosmetic containing one or two or more waxes selected from the above-mentioned plant-based waxes that are solid at 25°C and animal-based waxes that are solid at 25°C, and the above-mentioned oil component that is liquid or pasty at 20°C.

That is, the hardness improver of the present embodiment is an agent for improving the hardness of oily solid cosmetics, and comprises an oligoester obtained by esterifying a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D). The esterification reaction is performed using 0.65 to 0.8 moles of the component (B) and a total amount of 1.2 to 1.7 moles of the components (C) and (D) per mole of the component (A), and a mass ratio of the component (C) to the component (D) used in the esterification reaction is component (C):component (D)=10:90 to 99.9:0.1, preferably component (C):component (D)=10:90 to 99:1, more preferably component (C):component (D)=25:75 to 99:1, even more preferably component (C):component (D)=40:60 to 99:1, still more preferably component (C):component (D)=40:60 to 70:30, most preferably component (C):component (D)=50:50 to 70:30, and also preferably component (C):component (D)=10:90 to 70:30. The oligoester has an acid value of 5 mgKOH/g or less, a saponification value of 420 to 510 mgKOH/g, and a hydroxyl value of 60 mgKOH/g or less. The hardness improver of the present embodiment improves the hardness of an oily solid cosmetic containing one or two or more waxes selected from plant-based waxes that are solid at 25°C and animal-based waxes that are solid at 25°C.

The components (A) to (D) and the reaction conditions for the esterification reaction thereof are the same as those for the oligoester of the component (3).

By mixing the hardness improver of the present embodiment into an oily solid cosmetic containing one or two or more waxes selected from plant-based waxes that are solid at 25°C and animal-based waxes that are solid at 25°C, the hardness of the resulting oily solid cosmetic can be made higher than the hardness of an oily solid cosmetic obtained by mixing castor oil into an oily solid cosmetic of the same composition. The plant-based waxes that are solid at 25°C and the animal-based waxes that are solid at 25°C may be the same as those listed for the component (1) above.

Furthermore, by mixing the hardness improver of the present embodiment into an oily solid cosmetic containing one or two or more waxes selected from plant-based waxes that are solid at 25°C and animal-based waxes that are solid at 25°C, and an oily component that is liquid or pasty at 20°C, the hardness of the resulting oily solid cosmetic can be made higher than the hardness of an oily solid cosmetic obtained by mixing castor oil with an oily solid cosmetic of the same composition. The plant-based wax that is solid at 25°C and the animal-based wax that is solid at 25°C may be the same as those listed for the component (1) above, and the oily component that is liquid or pasty at 20°C may be the same as those listed for the component (2) above.

### Examples

The present invention will be described in more detail below with reference to Examples. It goes without saying that the scope of the present invention is not limited to these Examples. In the following Examples, parts and % represent parts by mass or % by mass unless otherwise specified.

In the following experiments, the acid value, saponification value, and hydroxyl value of the esterification reaction products were measured in accordance with the Standards for Quasi-drug Ingredients 2006.

In the following experiments, the waxes (plant-based waxes that are solid at 25°C or animal-based waxes that are solid at 25°C) as the component (1) and the oily components (oily components that are liquid or pasty at 20°C) as the component (2) that are relatively frequently used are shown in Table 1. For the components (1) and (2) that are not listed in Table 1, the trade names, manufacturers, or distributors are listed in each Example.

**[Table 1]**

| Table 1. Components (1) and (2) | | | | |
|---|---|---|---|---|
| Component | Name | Product name | Manufacturer or distributor | Melting point (°C) |
| (1) | Carnauba wax | CARNAUBA WAX TYPE1 | Pontes | 50°C or more |
| | Rice bran wax | Rice Wax SS-2 | Boso Oils & Fats | 50°C or more |
| | Candelilla wax | Refined Candelilla Wax SR-3 | Natural Products | 50°C or more |
| | Beeswax | Deodorizing Konjirusi bleached beeswax | Miki Chemical Industry Co., Ltd. | 50°C or more |
| | Sunflower seed wax | Refined Sunflower Wax | Yokozeki Oil & Fat Industries Co., Ltd. | 50°C or more |
| (2) | Castor oil | Castor oil | Fujifilm Wako Pure Chemical Industries, Ltd. | - |
| | Diisostearyl malate | Cosmol 222 | The Nisshin OilliO Group, Ltd. | - |
| | Polyglyceryl-2 triisostearate | Cosmol 43V | The Nisshin OilliO Group, Ltd. | - |
| | Polyglyceryl-10 decaisostearate | S Face IS-1009P | Sakamoto Pharmaceutical Co., Ltd. | - |
| | Caprylic/Capric triglyceride | O.D.O | The Nisshin OilliO Group, Ltd. | - |
| | Macadamia seed oil | Refined macadamia nut oil | Yokozeki Oil & Fat Industries Co., Ltd. | - |
| | Propanediol Dicaprylate/caprate | Salacos PR-85 | The Nisshin OilliO Group, Ltd. | |

### [Synthesis Example 1] Preparation of oligoester

A 1L four-neck flask equipped with a stirrer, a thermometer, a nitrogen gas inlet tube, and a water separator was charged with 191.9 g of glycerin, 172.3 g of succinic acid (purity: 99.5%, trade name: "Succinic Acid", manufactured by Fujifilm Wako Pure Chemical Industries, Ltd.), 291.5 g of caprylic acid (purity: 99% by mass or more, trade name: "PALMAC99-08", manufactured by IOI Acid chem), and 194.3 g of capric acid (purity: 99% by mass or more, trade name: "PALMAC99-10", manufactured by IOI Acid chem), and the mixture was heated to 230 to 240°C under a nitrogen stream. The esterification reaction was carried out for approximately 20 hours while removing the generated water. After completion of the reaction, excess acid was removed to obtain the desired esterified product, oligoester ((caprylic acid/capric acid/succinic acid) triglyceride).

The resulting oligoester had an acid value of 0.7 mgKOH/g, a saponification value of 461, and a hydroxyl value of 6.3 mgKOH/g.

### [Synthesis Examples 2 to 4] Preparation of oligoesters

Using the molar amounts and charged amounts shown in Table 2, an esterification reaction was carried out in the same manner as in the preparation of the oligoester in Synthesis Example 1, to prepare the oligoesters of Synthesis Examples 2 to 4.

**[Table 2]**

| Table 2. Charge amounts for esterification reaction | | | | | | | | | |
|---|---|---|---|---|---|---|---|---|---|
| | | Synthesis Example 1 | | Synthesis Example 2 | | Synthesis Example 3 | | Synthesis Example 4 | |
| Component | Component | Mass [g] | Molar amount | Mass [g] | Molar amount | Mass [g] | Molar amount | Mass [g] | Molar amount |
| A | Glycerin | 191.9 | 1.00 | 173.4 | 1.00 | 177.7 | 1.00 | 179.2 | 1.00 |
| B | Succinic acid | 172.3 | 0.70 | 166.8 | 0.75 | 182.3 | 0.80 | 126.4 | 0.55 |
| C | Caprylic Acid | 291.5 | 0.97 | 245.9 | 0.91 | 39.0 | 0.14 | 326.6 | 1.16 |
| D | Capric Acid | 194.3 | 0.54 | 163.9 | 0.51 | 351.0 | 1.06 | 217.8 | 0.65 |
| Mass ratio of component (C) to component (D) [(C):(D)] | | 60:40 | | 60:40 | | 10:90 | | 60:40 | |
| Acid value [mg KOH/g] | | 0.7 | | 1.0 | | 1.7 | | 1.1 | |
| Saponification value [mg KOH/g] | | 461 | | 467 | | 452 | | 425 | |
| Hydroxyl value [mg KOH/g] | | 6.3 | | 1.4 | | 1.2 | | 10.9 | |

### <Evaluation of oligoesters>

The oxidation stability of the oligoester synthesized in Synthesis Example 1 was evaluated by a CDM test (standard fat and oil test method: 120°C, air: 20 L/h). In the test, the conductivity was measured over time using a Rancimat 743 (manufactured by Metrohm) device. In the CDM test, the time to the inflection point where the conductivity increases rapidly over time (induction time) is measured. The longer the induction time, the better the oxidation stability, and the one where no significant increase in conductivity is observed has the best oxidation stability.

**[Table 3]**

| Table 3. Evaluation results of oxidation stability | |
|---|---|
| Component | Induction time |
| Castor oil [%] | 34.4 hours |
| Diisostearyl malate [%] | 16.8 hours |
| Synthesis Example 1 [%] | No significant increase in conductivity is observed |

The evaluation results are shown in Table 3. The oligoester of Synthesis Example 1 showed improved oxidation stability compared to castor oil and diisostearyl malate. Furthermore, the oligoester of Synthesis Example 1 showed no change in color before and after the test, and the change in odor was slight. Castor oil showed a yellowing of its color before and after the test, and emitted a deterioration odor, clearly indicating poor oxidation stability.

### [Examples 1 to 9, Comparative Examples 1 to 7]

Oily solid cosmetics were produced according to the formulations shown in Tables 5 and 6, and a test of the solidification ability was carried out when combined with a plant-based wax or an animal-based wax.

### <Preparation of oily solid cosmetics>

Based on the formulations in Tables 5 and 6, all of the raw material ingredients were stirred and mixed in a heated state at 90°C, poured into a polypropylene container (diameter: 36 mm, depth: 11 mm), and cooled to room temperature to prepare cylindrical oily solid cosmetics.

### <Hardness evaluation>

A gel hardness tester (product name: "Small Tabletop Tester EZ-Test EZ-SX", manufactured by Shimadzu Corporation) was used. A flat plunger with a diameter of 3 mm was pressed 0.4 mm into the oily solid cosmetic in the polycarbonate container after it had been left standing overnight in a thermostatic bath at 20°C. The maximum stress (gf) was recorded as the hardness of the oily solid cosmetic.

### <Evaluation of hardness-improving effect>

The hardness of oily solid cosmetics varies depending on the plant-based wax or animal-based wax used. Therefore, to evaluate the hardness-improving effect of the oligoester blended in the produced oily solid cosmetics, the hardness of the oily solid cosmetics made with castor oil and various plant-based waxes or animal-based waxes was measured, and the hardness was set to 1 to calculate the relative hardness value.

The hardness-improving effect was evaluated according to the following evaluation criteria. When the evaluation was "a" or "b", it was determined that the hardness-improving effect was high.

Evaluation criteria for hardness-improving effect:
a: Relative hardness value was 1.05 or more.
b: Relative hardness value was 0.95 or more and less than 1.05.
c: Relative hardness value was less than 0.95.

Table 4 shows the hardness (gf) (basic data) of the oily solid cosmetics used as the standard for the hardness-improving effect.

**[Table 4]**

| Table 4. Basic data | | | | | | |
|---|---|---|---|---|---|---|
| Classification | Component | Basic data 1 | Basic data 2 | Basic data 3 | Basic data 4 | Basic data 5 |
| (1) | Carnauba wax [%] | 20 | - | - | - | - |
| | Rice bran wax [%] | - | 20 | - | - | - |
| | Candelilla wax [%] | - | - | 20 | - | - |
| | Beeswax [%] | - | - | - | 20 | - |
| | Sunflower seed wax [%] | - | - | - | - | 20 |
| (2) | Castor oil [%] | 80 | 80 | 80 | 80 | 80 |
| Total [%] | | 100 | 100 | 100 | 100 | 100 |
| Hardness [gf] | | 615 | 464 | 1476 | 901 | 1297 |

### <Evaluation of oil bleeds>

The oily bleeds of the produced oily solid cosmetics were evaluated. Specifically, pressure was applied to the measurement sample with the index finger, and the seepage of liquid oil around the area where pressure was applied was evaluated. Those that seep liquid oil upon being pressed tend to cause a deterioration in the feeling of use and cause smudging of the makeup. When the evaluation was "a" or "b", it was determined to have a high oil bleed suppression effect.

Evaluation criteria for oil bleed:
a: Liquid oil did not seep out even when pressurized.
b: A small amount of liquid oil seeped out when pressurized.
c: Liquid oil clearly seeped out when pressurized.

**[Table 5]**

| Table 5. Evaluation results | | | | | | | | | | | |
|---|---|---|---|---|---|---|---|---|---|---|---|
| Classification | Component | Com. Example 1 | Com. Example 2 | Com. Example 3 | Com. Example 4 | Com. Example 5 | Example 1 | Example 2 | Example 3 | Example 4 | Example 5 |
| (1) | Carnauba wax [%] | 20 | - | - | - | - | 20 | - | - | - | - |
| | Rice bran wax [%] | - | 20 | - | - | - | - | 20 | - | - | - |
| | Candelilla wax [%] | - | - | 20 | - | - | - | - | 20 | - | - |
| | Beeswax [%] | - | - | - | 20 | - | - | - | - | 20 | |
| | Sunflower seed wax [%] | - | - | - | - | 20 | - | - | - | - | 20 |
| (2) | Diisostearyl malate [%] | 80 | 80 | 80 | 80 | 80 | - | - | - | - | - |
| (3) | Synthesis Example 1 [%] | - | - | - | - | - | 80 | 80 | 80 | 80 | 80 |
| Total [%] | | 100 | 100 | 100 | 100 | 100 | 100 | 100 | 100 | 100 | 100 |
| Hardness [gf] | | 473 | 19.6 | 1147 | 90 | 1218 | 763 | 586 | 1856 | 900 | 1317 |
| Relative hardness values when the hardness (basic data) of the plant-based waxes or the animal-based waxes and castor oil was set to 1 | | 0.77 | 0.04 | 0.78 | 0.10 | 0.94 | 1.24 | 1.26 | 1.26 | 1.00 | 1.02 |
| Hardness-improving effect evaluation | | c | c | c | c | c | a | a | a | b | b |
| Oil bleed evaluation | | a | c | a | c | a | a | a | a | a | a |

**[Table 6]**

| Table 6. Evaluation results | | | | | | | |
|---|---|---|---|---|---|---|---|
| Classification | Component | Comparative Example 6 | Comparative Example 7 | Example 6 | Example 7 | Example 8 | Example 9 |
| (1) | Carnauba wax [%] | - | - | 20 | - | 20 | - |
| | Rice bran wax [%] | - | - | - | 20 | - | 20 |
| | Candelilla wax [%] | 20 | - | - | - | - | - |
| | Beeswax [%] | - | 20 | - | - | - | 20 |
| (2) | Synthesis Example 4 [%] | 80 | 80 | - | - | - | - |
| (3) | Synthesis Example 2 [%] | - | - | 80 | 80 | | |
| (3) | Synthesis Example 3 [%] | - | - | - | - | 80 | 80 |
| Total [%] | | 100 | 100 | 100 | 100 | 100 | 100 |
| Hardness [gf] | | 1106 | 582 | 752 | 543 | 854 | 534 |
| Relative hardness values when the hardness (basic data) of the plant-based waxes or the animal-based waxes and castor oil is set to 1 | | 0.75 | 0.65 | 1.22 | 1.17 | 1.39 | 1.15 |
| Hardness-improving effect evaluation | | c | c | a | a | a | a |
| Oil bleed evaluation | | a | a | a | a | a | a |

The evaluation results for each oily solid cosmetic are shown in Tables 5 and 6. The oily solid cosmetics prepared using the oligoesters of Synthesis Examples 1 to 3 and a plant-based wax or animal-based wax showed a good hardness-improving effect and were resistant to oil bleeding. It has been known that castor oil can be combined with a plant-based wax or animal-based wax to prepare an oily solid cosmetic with high hardness and good shape retention, but the oligoesters of Synthesis Examples 1 to 3 demonstrated even better performance (Examples 1 to 9).

On the other hand, the relative hardness values of the oily solid cosmetics in which diisostearyl malate was blended instead of the oligoester of Synthesis Example 1 were all less than 1, indicating that blending diisostearyl malate had no effect on improving hardness, and when combined with rice bran wax, beeswax, etc., the hardness was extremely low (Comparative Examples 1 to 5).

Furthermore, the relative hardness values of the oily solid cosmetics in which the oligoester of Synthesis Example 4 corresponding to the component (2) was blended instead of the oligoester of Synthesis Example 1 and candelilla wax or beeswax was used as the wax were all less than 1, indicating that blending the oligoester of Synthesis Example 4 had no effect on improving hardness (Comparative Examples 6 and 7).

### [Examples 10 to 27, Comparative Examples 8 to 10] Lip balm

Using the oligoesters of Synthesis Examples 1 to 3, lip balms, which are oily solid cosmetics, were prepared.

<Preparation of lip balm>

Based on the formulations in Tables 7 to 10, all raw ingredients were mixed and stirred at 90°C, poured into a polypropylene container (diameter: 36 mm, depth: 11 mm), and cooled to room temperature to prepare cylindrical lip balms. These cylindrical lip balms were used for hardness measurements and oil bleed evaluations.

### <Hardness evaluation>

A gel hardness tester (product name: "Small Tabletop Tester EZ-Test EZ-SX", manufactured by Shimadzu Corporation) was used. A flat plunger with a diameter of 3 mm was pressed 0.4 mm into the cylindrical lip balm in the polycarbonate container after it had been left standing overnight in a thermostatic chamber at 20°C. The maximum stress (gf) was recorded as the hardness of the lip balm.

The hardness was evaluated according to the following evaluation criteria. When the evaluation was "a", "b", or "c", it was determined that the hardness was sufficiently high and that the shape retention was high.

Hardness evaluation criteria:
a: The hardness was 300gf or more.
b: The hardness was 250 gf or more and less than 300 gf.
c: The hardness was 200 gf or more and less than 250 gf.
d: The hardness was 150 gf or more and less than 200 gf.
e: The hardness was 150 gf or less.

### <Evaluation of oil bleeds>

The oily bleeds of the prepared oily solid cosmetics were evaluated. Specifically, pressure was applied to the measurement sample with the index finger, and the seepage of liquid oil around the area where pressure was applied was evaluated. Those that seep liquid oil upon being pressed tended to cause a deterioration in the feeling of use and cause smudging of the makeup. When the evaluation was "a" or "b", it was determined to have a high oil bleed suppression effect.

Evaluation criteria for oil bleeds:
a: Liquid oil did not seep out even when pressurized.
b: A small amount of liquid oil seeped out when pressurized.
c: Liquid oil clearly seeped out when pressurized.

**[Table 7]**

| Table 7. Lip balm | | | | | | | |
|---|---|---|---|---|---|---|---|
| Classification | Component | Example 10 | Example 11 | Example 12 | Example 13 | Example 14 | Comparative Example 8 |
| (1) | Carnauba wax [%] | 20 | 20 | 20 | 20 | 20 | 20 |
| (2) | Polyglyceryl-2 triisostearate [%] | 10 | 20 | 40 | - | - | 80 |
| | Polyglyceryl-10 decaisostearate [%] | - | - | - | 40 | - | - |
| | Caprylic/Capric triglyceride [%] | - | - | - | - | 40 | - |
| (3) | Synthesis Example 1 [%] | 70 | 60 | 40 | 40 | 40 | - |
| Total [%] | | 100 | 100 | 100 | 100 | 100 | 100 |
| Total amount of component (1) | | 20 | 20 | 20 | 20 | 20 | 20 |
| Total amount of component (2) | | 10 | 20 | 40 | 40 | 40 | 80 |
| Component (3)/(Component (2)+Component (3)) | | 0.9 | 0.8 | 0.5 | 0.5 | 0.5 | 0.0 |
| Effects | Hardness [gf] | 411 | 396 | 273 | 356 | 238 | 173 |
| | Hardness evaluation | a | a | b | a | c | d |
| | Oil bleed evaluation | a | a | a | a | a | c |

**[Table 8]**

| Table 8. Lip balm | | | | | | | | | |
|---|---|---|---|---|---|---|---|---|---|
| Classification | Component | Example 15 | Example 16 | Example 17 | Example 18 | Example 19 | Example 20 | Example 21 | Example 22 |
| (1) | Carnauba wax [%] | 20 | 20 | 20 | 20 | 20 | 20 | 20 | 20 |
| (2) | Polyglyceryl-2 triisostearate [%] | 20 | 40 | - | - | 20 | 40 | - | - |
| | Polyglyceryl-10 decaisostearate [%] | - | - | 40 | - | - | - | 40 | - |
| | Caprylic/Capric triglyceride [%] | - | - | - | 40 | - | - | - | 40 |
| (3) | Synthesis Example 2 [%] | 60 | 40 | 40 | 40 | - | - | - | - |
| | Synthesis Example 3 [%] | - | - | - | - | 60 | 40 | 40 | 40 |
| Total [%] | | 100 | 100 | 100 | 100 | 100 | 100 | 100 | 100 |
| Total amount of component (1) | | 20 | 20 | 20 | 20 | 20 | 20 | 20 | 20 |
| Total amount of component (2) | | 20 | 40 | 40 | 40 | 20 | 40 | 40 | 40 |
| Component (3)/ (Component (2)+Component (3)) | | 0.8 | 0.5 | 0.5 | 0.5 | 0.8 | 0.5 | 0.5 | 0.5 |
| Effects | Hardness [gf] | 477 | 307 | 405 | 255 | 577 | 356 | 430 | 239 |
| | Hardness evaluation | a | a | a | b | a | a | a | c |
| | Oil bleed evaluation | a | a | a | b | a | a | a | b |

**[Table 9]**

| Table 9. Lip balm | | | | | | |
|---|---|---|---|---|---|---|
| Classification | Component | Example 23 | Example 24 | Example 25 | Comparative Example 9 | Comparative Example 10 |
| (1) | Rice bran wax [%] | 20 | - | - | 20 | - |
| | Beeswax [%] | - | 10.5 | 20 | - | 12 |
| (2) | Castor oil [%] | 20 | - | - | 20 | - |
| | Macadamia seed oil [%] | - | - | 48 | - | - |
| | Polyglyceryl-2 triisostearate [%] | 2 | - | 10 | 49 | - |
| | Polyglyceryl-10 decaisostearate [%] | 6 | - | - | 4 | 88 |
| | Caprylic/Capric triglyceride [%] | 2 | - | - | 2 | - |
| (3) | Synthesis Example 1 [%] | 50 | 89.5 | 22 | 5 | |
| Total [%] | | 100 | 100 | 100 | 100 | 100 |
| Total amount of component (1) | | 20 | 10.5 | 20 | 20 | 12 |
| Total amount of component (2) | | 30 | 0 | 58 | 75 | 88 |
| Component (3)/(Component (2)+Component (3)) | | 0.6 | 1.0 | 0.3 | 0.1 | 0.0 |
| Effects | Hardness [gf] | 329 | 336 | 500 | 74 | 36 |
| | Hardness evaluation | a | a | a | e | e |
| | Oil bleed evaluation | a | a | a | c | c |

**[Table 10]**

| Table 10. Lip balm | | | |
|---|---|---|---|
| Classification | Component | Example 26 | Example 27 |
| (1) | Candelilla wax [%] | 14 | - |
| | Sunflower seed wax [%] | - | 10 |
| (2) | Macadamia seed oil [%] | 48 | - |
| | Polyglyceryl-2 triisostearate [%] | 2 | 20 |
| | Polyglyceryl-10 decaisostearate [%] | 2 | 20 |
| | Caprylic/Capric triglyceride [%] | 2 | 20 |
| (3) | Synthesis Example 1 [%] | 32 | 30 |
| Total [%] | | 100 | 100 |
| Total amount of component (1) | | 14 | 10 |
| Total amount of component (2) | | 54 | 60 |
| Component (3)/(Component (2)+Component (3)) | | 0.4 | 0.3 |
| Effects | Hardness [gf] | 309 | 273 |
| | Hardness evaluation | a | b |
| | Oil bleed evaluation | a | a |

The evaluation results of the lip balms are shown in Tables 7 to 10. The lip balms of Examples 10 to 27 showed good hardness and resistance to oil bleeding. It was found that the hardness-improving effect and oil bleed-suppressing effect achieved by combining the oligoesters of Synthesis Examples 1, 2, and 3 with a plant-based wax or an animal-based wax are not significantly affected by other ingredients, and will likely be similarly exhibited in cosmetics with various compositions.

### [Example 28, Comparative Examples 11 and 12] Stick-type lip balm

Using the oligoesters of Synthesis Examples 1 to 3, stick-type lip balms, which are oily solid cosmetics, were prepared.

### <Preparation of lip balm>

Based on the formulation in Table 11, all raw materials were mixed and stirred at 90°C, poured into a bullet-shaped lipstick mold, and cooled to room temperature to obtain a stick-shaped lip balm. At the same time, the mixed and stirred raw materials were poured into a polypropylene container (diameter: 36 mm, depth: 11 mm) and cooled to room temperature to prepare a cylindrical lip balm. The stick-shaped lip balm was used for evaluating the feel of use. The cylindrical lip balm was also used for measuring hardness and evaluating oil bleeds.

In Table 11, the 1,2-hexanediol was "KMO-6" manufactured by Kankosha, and the caprylyl glycol was "OC-Diol" manufactured by Ochem Trading Co., Ltd.

### <Hardness evaluation>

The hardness of each lip balm was evaluated in the same manner as in Example 10.

### <Evaluation of oil bleeds>

The oily bleeding of each lip balm was evaluated in the same manner as in Example 10.

### <Evaluation of feel upon application>

A panel of five experts compared each lip balm against commercially available lip balms in terms of "resistance to oil bleeds", "adhesion", "spreadability", and "glossiness" using a three-point scale: "excellent", "equal", or "inferior". The results are shown in Table 11.

Evaluation criteria for feel upon application:
a: More than three out of five people rated it as "excellent" compared to commercially available lip balms.
b: Neither "a" nor "c" was applied.
c: Three or more out of five people rated it as "inferior" to commercially available lip balms.

**[Table 11]**

| Table 11. Lip balm | | | | |
|---|---|---|---|---|
| Classification | Component | Example 28 | Comparative Example 11 | Comparative Example 12 |
| (1) | Candelilla wax [%] | 4 | 4 | 4 |
| | Sunflower seed wax [%] | 6 | 6 | 6 |
| | Carnauba wax [%] | 4 | 4 | 4 |
| | Rice bran wax [%] | 2 | 2 | 2 |
| | Beeswax [%] | 2 | 2 | 2 |
| (2) | Polyglyceryl triisostearate-2 [%] | 39 | 39 | 39 |
| | Caprylic/Capric triglyceride [%] | - | 42 | - |
| | Propanediol Dicaprylate/caprate [%] | - | - | 42 |
| (3) | Synthesis Example 1 [%] | 42 | - | - |
| (4) | 1,2-Hexanediol [%] | 0.5 | 0.5 | 0.5 |
| | Caprylyl glycol (1,2-octanediol) [%] | 0.5 | 0.5 | 0.5 |
| Total [%] | | 100 | 100 | 100 |
| Total amount of component (1) | | 18 | 18 | 18 |
| Total amount of component (2) | | 39 | 81 | 81 |
| Component (3)/(Component (2)+Component (3)) | | 0.5 | 0 | 0 |
| Evaluation | Hardness [gf] | 314 | 122 | 113 |
| | Hardness evaluation | a | e | e |
| | Resistance to oil bleeds | b | c | c |
| | Adhesion | a | a | a |
| | Spreadability | a | a | a |
| | Glossiness | a | a | a |

The evaluation results of each lip balm are shown in Table 11. The lip balm of Example 28 showed sufficient hardness as a stick-type cosmetic product, caused little oily bleeding during use, and showed a good feel upon application. In addition, there was no problem with the preservative performance.

### [Example 29] Lipstick (stick-type)

Using the oligoester of Synthesis Example 1, a stick-type lipstick, which is an oily solid cosmetic, was produced.

### <Preparation of lipstick>

Based on the formulation in Table 12, all raw materials were mixed and stirred at 90°C until they were thoroughly homogenized, and then the mixture was degassed under reduced pressure and poured into a mold. The mold was then cooled to 20°C to obtain a lipstick (stick-type).

In Table 11, the pentaerythrityl tetraisostearate was "Salacos 5418V" manufactured by The Nisshin OilliO Group, Ltd., the dipentaerythrityl hexa(hydroxystearate/stearic acid/rosinate) was "Cosmol 168ARV" manufactured by The Nisshin OilliO Group, Ltd., the glyceryl ethylhexanoate/stearate/adipate was "Nomcort LAH" manufactured by The Nisshin OilliO Group, Ltd., the Phytosteryl/octyldodecyl lauroyl glutamate was "Eldew PS-203" manufactured by Ajinomoto Co., Inc., the sorbitan sesquiisostearate was "Cosmol 182V" manufactured by The Nisshin OilliO Group, Ltd., and the caprylyl glycol was "OC-Diol" manufactured by Ochem Trading Co., Ltd.

**[Table 12]**

| Table 12. Ingredients | Example 29 |
|---|---|
| Pentaerythrityl tetraisostearate [%] | 16 |
| Synthesis Example 1 [%] | 25.5 |
| Diisostearyl malate [%] | 12 |
| Polyglyceryl-2 triisostearate [%] | 10 |
| Dipentaerythrityl hexa(hydroxystearate/stearic acid/rosinate) [%] | 4 |
| Glyceryl Ethylhexanoate/Stearate/Adipate [%] | 2 |
| Phytosteryl/Octyldodecyl lauroyl glutamate [%] | 5 |
| Sorbitan sesquiisostearate [%] | 2 |
| Caprylyl glycol [%] | 0.5 |
| Candelilla wax [%] | 6 |
| Sunflower seed wax [%] | 6 |
| Carnauba wax [%] | 2 |
| Rice bran wax [%] | 2 |
| Red No. 201 [%] | 1 |
| Red No. 202 [%] | 2 |
| Titanium mica [%] | 4 |
| Total [%] | 100 |

The lipstick thus prepared showed sufficient hardness for a stick-type cosmetic, and showed good adhesion, spreadability, and glossiness with little oil bleeding during use. It also provided long-lasting moisturizing sensation and good makeup wear.

### Example 30 Lipstick (stick-type)

Using the oligoester of Synthesis Example 1, a stick-type lipstick, which is an oily solid cosmetic, was prepared.

### <Preparation of lipstick>

Based on the formulation in Table 13, a lipstick (stick-type) was obtained in the same manner as in Example 29.

In Table 13, the neopentyl glycol dicaprate was "Estemol N-01" manufactured by The Nisshin OilliO Group, Ltd., the phenyl trimethicone was "SH556 Fluid" manufactured by Toray Dow Corning, the dipentaerythrityl pentaisostearate was "Salacos DP-518N" manufactured by The Nisshin OilliO Group, Ltd., the hydrogenated polyisobutene was "Pearleam 18" manufactured by NOF Corp., the octyldodecanol was "N-Jecol 200A" manufactured by New Japan Chemical Co., Ltd., the dipentaerythrityl hexahydroxystearate was "Cosmol 168M" manufactured by The Nisshin OilliO Group, Ltd., the dipentaerythrityl tetra(hydroxystearate/isostearate) was "Cosmol 168EV" manufactured by The Nisshin OilliO Group, Ltd., the dipentaerythrityl tri-polyhydroxystearate was "Salacos WO-6" manufactured by The Nisshin OilliO Group, Ltd., the bis-behenyl/isostearyl/phytosteryl dimer dilinoleyl dimer dilinoleate was "Plandool-G" manufactured by Nippon Fine Chemicals, the caprylyl glycol was "OC-Diol" manufactured by Ochem Trading Co., Ltd, and the behenyl behenate was "Nomcort NW" manufactured by The Nisshin OilliO Group, Ltd.

**[Table 13]**

| Table 13. Raw materials | Example 30 |
|---|---|
| Synthesis Example 1 [%] | 15 |
| Neopentyl glycol dicaprate [%] | 23 |
| Phenyl trimethicone [%] | 10 |
| Dipentaerythrityl pentaisostearate [%] | 4 |
| Hydrogenated polyisobutene [%] | 2 |
| Diisostearyl malate [%] | 8 |
| Octyldodecanol [%] | 5 |
| Dipentaerythrityl hexahydroxystearate [%] | 4 |
| Dipentaerythrityl tetra(hydroxystearate/isostearate) [%] | 2 |
| Dipentaerythrityl tri-polyhydroxystearate [%] | 1 |
| Bis-behenyl/isostearyl/phytosteryl dimer dilinoleyl dimer dilinoleate [%] | 1 |
| Caprylyl glycol [%] | 0.5 |
| Candelilla wax [%] | 6 |
| Sunflower seed wax [%] | 8 |
| Carnauba wax [%] | 2 |
| Rice bran wax [%] | 2 |
| Behenyl behenate [%] | 2 |
| Red No. 201 [%] | 0.5 |
| Red No. 202 [%] | 2 |
| Titanium dioxide [%] | 2 |
| Total [%] | 100 |

The prepared lipstick showed sufficient hardness as a stick-type cosmetic, and showed good adhesion, spreadability, and glossiness with little oily bleeding during use.

### [Example 31] Oil-based foundation

The oligoester of Synthesis Example 1 was used to prepare an oil-based foundation, which is an oily solid cosmetic.

### <Preparation of oil-based foundation>

Based on the formulation in Table 14, an oil-based foundation was obtained in the same manner as in Example 29, except that the mold was replaced with a metal dish.

In Table 14, the mineral oil was "Carnation" manufactured by SONNEBORN, the cetyl ethylhexanoate was "Salacos 816T" manufactured by The Nisshin OilliO Group, Ltd., and the caprylyl glycol was "OC-Diol" manufactured by Ochem Trading Co., Ltd.

**[Table 14]**

| Table 14. Raw materials | Example 31 |
|---|---|
| Synthesis Example 1 [%] | 10 |
| Mineral oil [%] | 15 |
| Cetyl ethylhexanoate [%] | 17.45 |
| Polyglyceryl-2 triisostearate [%] | 5 |
| Beeswax [%] | 1 |
| Candelilla wax [%] | 6 |
| Sunflower seed wax [%] | 2 |
| Rice bran wax [%] | 2 |
| Caprylyl glycol [%] | 0.5 |
| Titanium dioxide [%] | 15 |
| Kaolin [%] | 10 |
| Talc [%] | 8 |
| Nylon powder [%] | 3 |
| Iron oxide (black) [%] | 0.05 |
| Iron oxide (yellow) [%] | 4 |
| Bengala [%] | 1 |
| Total [%] | 100 |

The oil-based foundation thus prepared showed sufficient shape retention as an oily solid cosmetic, and showed good adhesion and spreadability with little oil bleeding during use. When the oil-based foundation was used, it provided a beautiful finish with a natural gloss, was resistant to shine, and showed excellent makeup longevity. It also showed a high concealing effect on blemishes and the like.

### [Example 32] Lip liner pencil

The oligoester of Synthesis Example 1 was used to prepare a lip liner pencil, which is an oily solid cosmetic.

### <Preparation of lip liner pencil>

Based on the formulation in Table 15, the materials were kneaded together, and all materials except the volatile oil were melted at 100°C and mixed by stirring. The volatile oil was added to the resulting mixture, and the mixture was mixed by stirring at 80°C, filled into a container, and molded into a core body.

In Table 15, the cyclopentasiloxane was "KF-995" manufactured by Shin-Etsu Chemical Co., Ltd., the isododecane was "Marukasol R" manufactured by Maruzen Petrochemical Co., Ltd., the sorbitan sesquiisostearate was "Cosmol 182V" manufactured by The Nisshin OilliO Group, Ltd., and the caprylyl glycol was "OC-Diol" manufactured by Ochem Trading Co., Ltd.

**[Table 15]**

| Table 15. Raw materials | Example 32 |
|---|---|
| Synthesis Example 1 [%] | 10 |
| Cyclopentasiloxane [%] | 15 |
| Isododecane [%] | 32.8 |
| Rice bran wax [%] | 22 |
| Sorbitan sesquiisostearate [%] | 1 |
| Caprylyl glycol [%] | 0.2 |
| Red No. 201 [%] | 1 |
| Red No. 202 [%] | 2 |
| Titanium dioxide [%] | 9.9 |
| Iron oxide (black) [%] | 0.1 |
| Iron oxide (Yellow) [%] | 4 |
| Bengala [%] | 2 |
| Total [%] | 100 |

The lip liner pencil thus prepared showed sufficient shape retention as a pencil-type cosmetic, and showed good adhesion, spreadability, and glossiness with little oily bleeding during use. The cosmetic also showed good makeup longevity.

### INDUSTRIAL APPLICABILITY

According to the present invention, it is possible to provide an oily solid cosmetic that contains a plant-based wax or an animal-based wax, has excellent shape retention, is effective in suppressing oil bleeds during use, and has a good feel upon application, and to provide a hardness improver that is useful for producing the oily solid cosmetic.

## Claims

1. An oily solid cosmetic comprising:
component (1): one or two or more waxes selected from plant-based waxes that are solid at 25°C and animal-based waxes that are solid at 25°C; and
component (3): an oligoester obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D), wherein
the esterification reaction is performed using 0.65 to 0.8 moles of the component (B) and a total amount of 1.2 to 1.7 moles of the components (C) and (D) per mole of the component (A),
a mass ratio of the component (C) to the component (D) used in the esterification reaction is component (C):component (D)=10:90 to 99.9, and
the oligoester has an acid value of 5 mgKOH/g or less; a saponification value of 420 to 510 mgKOH/g; and a hydroxyl value of 60 mgKOH/g or less.

2. The oily solid cosmetic according to claim 1, wherein the component (1) is one or two or more waxes selected from the group consisting of a carnauba wax, a rice bran wax, a candelilla wax, a sunflower seed wax, and a beeswax.

3. The oily solid cosmetic according to claim 1, wherein the plant-based wax is one or two or more selected from the group consisting of a carnauba wax, a rice bran wax, a candelilla wax, and a sunflower seed wax.

4. The oily solid cosmetic according to claim 1, wherein the oily solid cosmetic does not contain any hydrocarbon wax (excluding plant-based waxes containing naturally derived hydrocarbon components and animal-based waxes containing naturally derived hydrocarbon components).

5. The oily solid cosmetic according to claim 1, wherein the component (B) is a succinic acid.

6. The oily solid cosmetic according to claim 1, wherein the component (C) is a caprylic acid.

7. The oily solid cosmetic according to claim 1, wherein the component (D) is a capric acid.

8. The oily solid cosmetic according to claim 1, wherein the component (B) is a succinic acid, the component (C) is a caprylic acid, and the component (D) is a capric acid.

9. The oily solid cosmetic according to claim 1, further comprising:
component (2): an oily component that is liquid or pasty at 20°C (excluding the component (3)).

10. The oily solid cosmetic according to claim 1, wherein
a content of the component (1) is 5 to 30% by mass, and a content of the component (3) is 10 to 90% by mass, relative to a total mass of the oily solid cosmetic.

11. The oily solid cosmetic according to claim 9, wherein
a content of the component (1) is 5 to 30% by mass, a content of the component (2) is more than 0% by mass but not more than 60% by mass, and a content of the component (3) is 10 to 90% by mass, relative to a total mass of the oily solid cosmetic.

12. The oily solid cosmetic according to claim 9, wherein a mass ratio of the component (3) to a total amount of the component (2) and the component (3) ([amount (g) of component (3)]/([amount (g) of component (2)]+[amount (g) of the component (3)]) is 0.2 to 1.0.

13. The oily solid cosmetic according to claim 1, further comprising:
component (4): one or two or more selected from the group consisting of a 1,2-hexanediol, a caprylyl glycol, an ethylhexylglycerin, and a phenoxyethanol.

14. The oily solid cosmetic according to any one of claims 1 to 13, wherein the oily solid cosmetic is a stick-type cosmetic.

15. A hardness improver for improving hardness of an oily solid cosmetic, comprising an oligoester obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D), wherein
the esterification reaction is performed using 0.65 to 0.8 moles of the component (B) and a total amount of 1.2 to 1.7 moles of the components (C) and (D) per mole of the component (A),
a mass ratio of the component (C) to the component (D) used in the esterification reaction is component (C):component (D)=10:90 to 99.9,
the oligoester has an acid value of 5 mgKOH/g or less; a saponification value of 420 to 510 mgKOH/g; and a hydroxyl value of 60 mgKOH/g or less, and
the oily solid cosmetic contains one or two or more waxes selected from plant-based waxes that are solid at 25°C and animal-based waxes that are solid at 25°C.

16. Use of an oligoester as a hardness improver for improving hardness of an oily solid cosmetic, wherein
the oligoester is obtained by esterification reaction of a glycerin as component (A), a divalent carboxylic acid having 2 to 12 carbon atoms as component (B), one or two or more fatty acids selected from the group consisting of a caprylic acid, an ethylhexanoic acid, an isononanoic acid, an isopalmitic acid, and an isostearic acid as component (C), and one or two or more fatty acids selected from linear saturated fatty acids having 10 to 24 carbon atoms as component (D),
the esterification reaction is performed using 0.65 to 0.8 moles of the component (B) and a total amount of 1.2 to 1.7 moles of the components (C) and (D) per mole of the component (A),
a mass ratio of the component (C) to the component (D) used in the esterification reaction is component (C):component (D)=10:90 to 99.9:0.1,
the oligoester has an acid value of 5 mgKOH/g or less; a saponification value of 420 to 510 mgKOH/g; and a hydroxyl value of 60 mgKOH/g or less, and
the oily solid cosmetic contains one or two or more waxes selected from plant-based waxes that are solid at 25°C and animal-based waxes that are solid at 25°C.
